(19) Europäisches Patentamt
European Patent Office
Office européen des brevets

(11) EP 2 117 542 B1

(12) **EUROPEAN PATENT SPECIFICATION**

(45) Date of publication and mention of the grant of the patent:
**17.08.2011 Bulletin 2011/33**

(21) Application number: **08702047.5**

(22) Date of filing: **06.02.2008**

(51) Int Cl.:
***A61K 31/428*** *(2006.01)*

(86) International application number:
**PCT/GB2008/000386**

(87) International publication number:
**WO 2008/096111 (14.08.2008 Gazette 2008/33)**

(54) **COMBINATIONS OF BETA- 2 -ADRENOCEPTOR AGONISTIC BENZOTHIAZOLONE**

KOMBINATIONEN AUS EINEM BETA-2-ADRENOZEPTOR-AGONIST UND BENZOTHIAZOLON

COMBINAISONS DE BENZOTHIAZOLONE AGONISTE DE L'ADRENORECEPTEUR BETA 2

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **08.02.2007 GB 0702456**

(43) Date of publication of application:
**18.11.2009 Bulletin 2009/47**

(60) Divisional application:
**11163020.8**

(73) Proprietor: **AstraZeneca AB**
**151 85 Södertälje (SE)**

(72) Inventors:
- **CADOGAN, Elaine Bridget**
  **Loughborough**
  **Leicestershire LE11 5RH (GB)**
- **CONNOLLY, Stephen**
  **Loughborough**
  **Leicestershire LE11 5RH (GB)**
- **NICHOLLS, David, John**
  **Loughborough**
  **Leicestershire LE11 5RH (GB)**
- **WILEY, Katherine, Elisabeth**
  **Loughborough**
  **Leicestershire LE11 5RH (GB)**
- **YOUNG, Alan**
  **Loughborough**
  **Leicestershire LE11 5RH (GB)**

(56) References cited:
**WO-A-2007/018461**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 117 542 B1

## Description

The present invention relates to a combination of two or more pharmaceutically active substances for use in the treatment of respiratory diseases (for example chronic obstructive pulmonary disease (COPD) or asthma).

The essential function of the lungs requires a fragile structure with enormous exposure to the environment, including pollutants, microbes, allergens, and carcinogens. Host factors, resulting from interactions of lifestyle choices and genetic composition, influence the response to this exposure. Damage or infection to the lungs can give rise to a wide range of diseases of the respiratory system (or respiratory diseases). A number of these diseases are of great public health importance. Respiratory diseases include Acute Lung Injury, Acute Respiratory Distress Syndrome (ARDS), occupational lung disease, lung cancer, tuberculosis, fibrosis, pneumoconiosis, pneumonia, emphysema, Chronic Obstructive Pulmonary Disease (COPD) and asthma.

Among the most common of the respiratory diseases is asthma. Asthma is generally defined as an inflammatory disorder of the airways with clinical symptoms arising from intermittent airflow obstruction. It is characterised clinically by paroxysms of wheezing, dyspnea and cough. It is a chronic disabling disorder that appears to be increasing in prevalence and severity. It is estimated that 15% of children and 5% of adults in the population of developed countries suffer from asthma. Therapy should therefore be aimed at controlling symptoms so that normal life is possible and at the same time provide basis for treating the underlying inflammation.

COPD is a term which refers to a large group of lung diseases which can interfere with normal breathing. Current clinical guidelines define COPD as a disease state characterized by airflow limitation that is not fully reversible. The airflow limitation is usually both progressive and associated with an abnormal inflammatory response of the lungs to noxious particles and gases. The most important contributory source of such particles and gases, at least in the western world, is tobacco smoke. COPD patients have a variety of symptoms, including cough, shortness of breath, and excessive production of sputum; such symptoms arise from dysfunction of a number of cellular compartments, including neutrophils, macrophages, and epithelial cells. The two most important conditions covered by COPD are chronic bronchitis and emphysema.

Chronic bronchitis is a long-standing inflammation of the bronchi which causes increased production of mucous and other changes. The patients' symptoms are cough and expectoration of sputum. Chronic bronchitis can lead to more frequent and severe respiratory infections, narrowing and plugging of the bronchi, difficult breathing and disability.

Emphysema is a chronic lung disease which affects the alveoli and/or the ends of the smallest bronchi. The lung loses its elasticity and therefore these areas of the lungs become enlarged. These enlarged areas trap stale air and do not effectively exchange it with fresh air. This results in difficult breathing and may result in insufficient oxygen being delivered to the blood. The predominant symptom in patients with emphysema is shortness of breath.

Therapeutic agents used in the treatment of respiratory diseases include corticosteroids. Corticosteroids (also known as glucocorticosteroids or glucocorticoids) are potent antiinflammatory agents. Whilst their exact mechanism of action is not clear, the end result of corticosteroid treatment is a decrease in the number, activity and movement of inflammatory cells into the bronchial submucosa, leading to decreased airway responsiveness. Corticosteroids may also cause reduced shedding of bronchial epithelial lining, vascular permeability, and mucus secretion. Whilst corticosteroid treatment can yield important benefits, the efficacy of these agents is often far from satisfactory, particularly in COPD. Moreover, whilst the use of steroids may lead to therapeutic effects, it is desirable to be able to use steroids in low doses to minimise the occurrence and severity of undesirable side effects that may be associated with regular administration. Recent studies have also highlighted the problem of the acquisition of steroid resistance amongst patients suffering from respiratory diseases. For example, cigarette smokers with asthma have been found to be insensitive to short term inhaled corticosteroid therapy, but the disparity of the response between smokers and non-smokers appears to be reduced with high dose inhaled corticosteroid (Tomlinson et al., Thorax 2005;60:282-287).

A further class of therapeutic agent used in the treatment of respiratory diseases are bronchodilators. Bronchodilators may be used to alleviate symptoms of respiratory diseases by relaxing the bronchial smooth muscles, reducing airway obstruction, reducing lung hyperinflation and decreasing shortness of breath. Types of bronchodilators in clinical use include $\beta_2$ adrenoceptor agonists, muscarinic receptor antagonists and methylxanthines. Bronchodilators are prescribed mainly for symptomatic relief and they are not considered to alter the natural history of respiratory diseases.

*N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide and its dihydrochloride and dihydrobromide salts are β2 adrenoceptor agonists and are disclosed in PCT/SE2006/000927 (published as WO 2007/018461, see Examples 7, 15 and 16). The compound and its salts show at least a 10-fold selectivity of β2 adrenoceptor agonism over adrenergic α1D, adrenergic β1 and dopamine D2 activities.

Combination products comprising a $\beta_2$ adrenoceptor agonist and a corticosteroid are available. One such product is a combination of budesonide and formoterol fumarate (marketed by AstraZeneca under the tradename Symbicort ®), which has proven to be effective in controlling asthma and COPD, and improving quality of life in many patients.

In view of the complexity of respiratory diseases such as asthma and COPD, it is unlikely that any one mediator

can satisfactorily treat the disease alone. Moreover, whilst combination treatments using a $\beta_2$ adrenoceptor agonist and a corticosteroid deliver significant patient benefits, there remains a medical need for new therapies against respiratory diseases such as asthma and COPD, in particular for therapies with disease modifying potential.

Accordingly, the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient which is a muscarinic antagonist which is Aclidinium bromide, Glycopyrrolate (such as R,R-, R,S-, S,R-, or S,S-glycopyrronium bromide), Oxitropium bromide, Pirenzepine, telenzepine or Tiotropium bromide.

In one particular aspect the present invention provides a pharmaceutical product wherein the first and second active ingredients are in forms suitable for oral administration (for example for delivery to the lungs and/or airways).

The pharmaceutical product of the present invention comprises a first active ingredient and a second active ingredient, and it may comprise a third active ingredient. The third active ingredient can be chosen from the list of second active ingredients but would normally have a different mechanism of action. So, for example, the third active ingredient might be: a non-steroidal glucocorticosteroid receptor agonist, corticosteroid, a CCR1 antagonist or a PDE4 inhibitor.

The first and second active ingredients can be administered simultaneously (either in a single pharmaceutical preparation {that is, the active ingredients are in admixture} or via separate preparations), or sequentially or separately via separate pharmaceutical preparations.

In one particular aspect the present invention provides a pharmaceutical product comprising the first and second active ingredients in admixture. Alternatively, the pharmaceutical product may, for example, be a kit comprising a preparation of the first active ingredient and a preparation of the second active ingredient and, optionally, instructions for the simultaneous, sequential or separate administration of the preparations to a patient in need thereof.

In one aspect of the invention the muscarinic receptor antagonist is a long acting muscarinic receptor antagonist, i.e. a muscarinic receptor antagonist with activity that persists for more than 12 hours. Examples of long acting muscarinic receptor antagonists include tiotropium bromide.

In another aspect the present invention provides a pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient is Tiotropium bromide.

The first active ingredient and the second active ingredient of the pharmaceutical product of the present invention may be administered simultaneously, sequentially or separately to treat respiratory diseases. By simultaneously is meant that the active ingredients are in admixture, or they could be in separate chambers of the same inhaler. By sequential it is meant that the active ingredients are administered, in any order, one immediately after the other. They still have the desired effect if they are administered separately, but when administered in this manner they are generally administered less than 4 hours apart, conveniently less than two hours apart, more conveniently less than 30 minutes apart and most conveniently less than 10 minutes apart, for example less than 10 minutes but not one immediately after the other.

The active ingredients of the present invention may be administered by oral or parenteral (e.g. intravenous, subcutaneous, intramuscular or intraarticular) administration using conventional systemic dosage forms, such as tablets, capsules, pills, powders, aqueous or oily solutions or suspensions, emulsions and sterile injectable aqueous or oily solutions or suspensions. The active ingredients may be delivered to the lung and/or airways via oral administration in the form of a solution, suspension, aerosol or dry powder formulation. These dosage forms will usually include one or more pharmaceutically acceptable ingredients which may be selected, for example, from an adjuvant, carrier, binder, lubricant, diluent, stabilising agent, buffering agent, emulsifying agent, viscosity-regulating agent, surfactant, preservative, flavouring or colorant. As will be understood by those skilled in the art, the most appropriate method of administering the active ingredients is dependent on a number of factors.

In another embodiment the first and second active ingredients are administered via a single pharmaceutical composition (that is, the first and second active ingredients are in admixture). Therefore, the present invention further provides a pharmaceutical composition comprising, in admixture, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient as defined above. The pharmaceutical composition optionally further comprises a pharmaceutically acceptable adjuvant, diluent or carrier.

The pharmaceutical compositions of the present invention can be prepared by mixing the first active ingredient with the second active ingredient and a pharmaceutically acceptable adjuvant, diluent or carrier. Therefore, in a further aspect of the present invention there is provided a process for the preparation of a pharmaceutical composition, which comprises mixing the first and second active ingredients and a pharmaceutically acceptable adjuvant, diluent or carrier.

It will be understood that the therapeutic dose of each active ingredient administered in accordance with the present invention will vary depending upon the particular active ingredient employed, the mode by which the active ingredient is to be administered, and the condition or disorder to be treated.

In one embodiment of the present invention, the first active ingredient is administered via inhalation. When

administered via inhalation the dose of the first active ingredient will generally be in the range of from 0.1 microgram (μg) to 5000 μg, 0.1 to 1000 μg, 0.1 to 500 μg, 0.1 to 100 μg, 0.1 to 50 μg, 0.1 to 5 μg, 5 to 5000 μg, 5 to 1000 μg, 5 to 500 μg, 5 to 100 μg, 5 to 50 μg, 5 to 10 μg, 10 to 5000 μg, 10 to 1000 μg, 10 to 500 μg, 10 to 100μ g, 10 to 50 μg, 20 to 5000 μg, 20 to 1000 μg, 20 to 500 μg, 20 to 100 μg, 20 to 50 μg, 50 to 5000 μg, 50 to 1000 μg, 50 to 500 μg, 50 to 100 μg, 100 to 5000 μg, 100 to 1000 μg or 100 to 500 μg. The dose will generally be administered from 1 to 4 times a day, conveniently once or twice a day, and most conveniently once a day.

In one embodiment of the present invention the second active ingredient is administered by inhalation. When administered via inhalation the dose of the second active ingredient will generally be in the range of from 0.1 microgram (μg) to 5000 μg, 0.1 to 1000 μg, 0.1 to 500 μg, 0.1 to 100 μg, 0.1 to 50 μg, 0.1 to 5 μg, 5 to 5000 μg, 5 to 1000 μg, 5 to 500 μg, 5 to 100 μg, 5 to 50 μg, 5 to 10 μg, 10 to 5000 μg, 10 to 1000 μg, 10 to 500 μg, 10 to 100 μg, 10 to 50 μg, 20 to 5000 μg, 20 to 1000 μg, 20 to 500 μg, 20 to 100 μg, 20 to 50 μg, 50 to 5000 μg, 50 to 1000 μg, 50 to 500 μg, 50 to 100 μg, 100 to 5000 μg, 100 to 1000 μg or 100 to 500 μg. The dose will generally be administered from 1 to 4 times a day, conveniently once or twice a day, and most conveniently once a day.

In another embodiment the present invention provides a pharmaceutical product wherein the molar ratio of first active ingredient to second active ingredient is from 1:1000 to 1000:1, such as from 1:100 to 100:1, for example from 1:50 to 50: 1, for example 1:20 to 20:1.

In one embodiment, the present invention provides a pharmaceutical product comprising, in combination, a first active ingredient as defined above, and a second active ingredient as defined above, wherein each active ingredient is formulated for inhaled administration. In a further aspect of this embodiment, the pharmaceutical product is in the form of a pharmaceutical composition comprising the first and second active ingredients in admixture, and which composition is formulated for inhaled administration.

The active ingredients of the present invention are conveniently delivered via oral administration by inhalation to the lung and/or airways in the form of a solution, suspension, aerosol or dry powder (such as an agglomerated or ordered mixture) formulation. For example a metered dose inhaler device may be used to administer the active ingredients, dispersed in a suitable propellant and with or without an additional excipient such as ethanol, a surfactant, lubricant or stabilising agent. A suitable propellant includes a hydrocarbon, chlorofluorocarbon or a hydrofluoroalkane (e.g. heptafluoroalkane) propellant, or a mixture of any such propellants, for example in a pressurised metered dose inhaler (pMDI). Preferred propellants are P134a and P227, each of which may be used alone or in combination with other another propellant and/or surfactant and/or other excipient. A nebulised aqueous suspension or, preferably, solution may also be employed, with or without a suitable pH and/or tonicity adjustment, either as a unit-dose or multi-dose formulation. A suitable device for delivering a dry powder is Turbuhaler®.

The pharmaceutical product of the present invention can, for example, be administered: via an inhaler having the first and second active ingredients in separate chambers of the inhaler such that on administration the active ingredients mix in either the mouthpiece of the inhaler or the mouth of a patient or both (for simultaneous use); or, where the first and second active ingredients are in separate inhalers, via separate inhalers (for separate or sequential use); or the first and second active ingredients are in admixture in an inhaler when the inhaler is supplied to a patient (for simultaneous use).

A dry powder inhaler may be used to administer the active ingredients, alone or in combination with a pharmaceutically acceptable carrier (such as lactose), in the later case either as a finely divided powder or as an ordered mixture. The dry powder inhaler may be single dose or multi-dose and may utilise a dry powder or a powder-containing capsule.

Metered dose inhaler, nebuliser and dry powder inhaler devices are well known and a variety of such devices is available.

The pharmaceutical product of the present invention may be used to treat diseases of the respiratory tract such as obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis, and chronic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus.

Accordingly, the present invention further provides a pharmaceutical product, kit or composition according to the invention for use in therapy.

The present invention further provides the use of a pharmaceutical product according to the invention in the manufacture of a medicament for the treatment of a respiratory disease, in particular chronic obstructive pulmonary disease or asthma, for example chronic obstructive pulmonary disease.

In a further aspect the present invention provides the use of a pharmaceutical product, kit or composition as hereinbefore described for the treatment of a respiratory disease, in particular chronic obstructive pulmonary disease or asthma, for example chronic obstructive pulmonary disease.

In the context of the present specification, the term "therapy" also includes "prophylaxis" unless there are specific indications to the contrary. The terms "therapeutic" and "therapeutically" should be construed accordingly. Prophylaxis is expected to be particularly relevant to the treatment of persons who have suffered a previous episode of, or are otherwise considered to be at increased risk of, the condition or disorder in question. Persons at risk of developing a particular condition or disorder generally include those having a family history of the condition or disorder, or those who have been identified by genetic testing or screening to be particularly susceptible to developing the condition or disorder.

## General Preparative Methods

There follow preparative methods for *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide (Preparations 1 and 2) and also assays and data showing the activity of this compound (called Compound A in the assays and Table 1 below).

$^1$H NMR spectra were recorded on a Varian *Inova* 400 MHz or a Varian *Mercury*-VX 300 MHz instrument. The central peaks of chloroform-*d* ($\delta_H$ 7.27 ppm), dimethylsulfoxide-$d_6$ ($\delta_H$ 2.50 ppm), acetonitrile-$d_3$ ($\delta_H$ 1.95 ppm) or methanol-$d_4$ ($\delta_H$ 3.31 ppm) were used as internal references. Column chromatography was carried out using silica gel (0.040-0.063 mm, Mercy). Unless stated otherwise, starting materials were commercially available. All solvents and commercial reagents were of laboratory grade and were used as received.

The following method was used for LC/MS analysis:

Instrument Agilent 1100; Column Waters Symmetry 2.1 x 30 mm; Mass APCI; Flow rate 0.7 ml/min; Wavelength 254 nm; Solvent A: water + 0.1% TFA; Solvent B: acetonitrile + 0.1% TFA ; Gradient 15-95%/B 8 min, 95% B 1 min.

Analytical chromatography was run on a Symmetry $C_{18}$-column, 2.1 x 30 mm with 3.5 μm particle size, with acetonitrile/water/0.1% trifluoroacetic acid as mobile phase in a gradient from 5% to 95% acetonitrile over 8 minutes at a flow of 0.7 ml/min.

The abbreviations or terms used in the examples have the following meanings:

SCX: Solid phase extraction with a sulfonic acid sorbent
HPLC: High performance liquid chromatography
DMF: N,N-Dimethylformamide

## Preparation 1

***N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide**

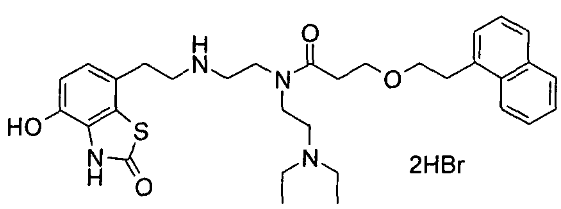

### a) *tert*-Butyl 3-[2-(1-naphthyl)ethoxy]propanoate

1-Naphthalene ethanol (10 g) was treated with benzyltrimethylammonium hydroxide (Triton B®; 0.9 mL of a 40% solution in methanol) and the resulting mixture stirred *in vacuo* for 30 minutes. The mixture was then cooled to 0˚C

and treated with tert-butyl acrylate (8.19 g). The resulting mixture was slowly warmed to room temperature and stirred overnight. The crude mixture was subsequently absorbed onto aluminium oxide (30 g) and eluted with diethylether (200 mL). The organics were concentrated to give a crude material (16.6 g) which was purified by flash silica chromatography eluting with 1:8, diethylether : hexane to give the subtitled compound (12.83 g).
$^1$H NMR ($CDCl_3$) $\delta$ 8.05 (dd, 1H), 7.84 (dd, 1H), 7.72 (dd, 1H), 7.54-7.34 (m, 4H), 3.81-3.69 (m, 4H), 3.35 (t, 2H), 2.52-2.47 (m, 2H), 1.45 (s, 9H).

**b) 3-[2-(1-Naphthyl)ethoxy]propanoic acid**

*tert*-Butyl 3-[2-(1-naphthyl)ethoxy]propanoate (6.19 g) was taken up in dichloromethane (30 mL) and treated with trifluoroacetic acid (5 mL). The resulting solution was stirred at room temperature for 2 hours, an additional 1 mL of trifluoroacetic acid was added and the solution stirred overnight. The mixture was concentrated, taken up in 2M sodium hydroxide solution (30 mL) and washed with ether (2 x 20 mL). The aqueous layer was subsequently acidified (using 1 M hydrochloric acid) and extracted with ether (2 x 30 mL). The combined organics were washed with brine (20 mL), dried over anhydrous magnesium sulphate, filtered and concentrated *in vacuo* to give the sub-titled compound (5.66 g) as a clear oil.
$^1$H NMR ($CDCl_3$) $\delta$ 8.05 (bs, I H), 7.85 (bs, I H), 7.74 (bs, 1H), 7.50-7.38 (m, 4H), 3.84-3.75 (bm, 4H), 3.39 (bs, 2H), 2.65 (bs, 2H).

**c) *N*-(2-Diethylaminoethyl)-*N*-(2-hydroxyethyl)-3-[2-(1-naphthyl)ethoxy]-propanamide**

Oxalyl chloride (0.33 g) was added dropwise to a solution of 3-[2-(1-naphthyl)ethoxy]propanoic acid (0.53 g) in dichloromethane (10 mL), dimethylformamide (1 drop) was added and stirring continued at room temperature for 1 hour. The mixture was subsequently concentrated, re-dissolved in dichloromethane (10 mL) and added dropwise to a solution of 2-(2-diethylaminoethylamino)ethanol (0.35 g) and diisopropylethylamine (0.56 g) in dichloromethane (10 mL). The resulting mixture was stirred at room temperature for 1 hour, diluted (dichloromethane, 50 mL), washed with water (2 x 20 mL), brine (20 mL), dried over magnesium sulfate and concentrated to give the crude product (0.91 g) which was purified by flash column chromatography (eluting with 5-7% methanol in dichloromethane) to give 0.63 g of the sub-titled compound.
$^1$H NMR ($CDCl_3$) $\delta$ 8.05 (d, 1H), 7.85 (d, 1H), 7.73 (d, 1H), 7.52-7.47 (m, 2H), 7.42-7.35 (m, 2H), 3.84-3.78 (m, 6H), 3.72-3.70 (m, 1/2H), 3.45-3.35 (m, 6H), 2.79-2.77 (m, 1+1/2H), 2.62-2.58 (m, 2H), 2.54-2.49 (m, 4H), 1.04-1.01 (m, 6H).

**d) *N*-[2-(Diethylamino)ethyl]-*N*-(2-{[2-(4-hydroxy-2-oxo-2,3-dihydro-1,3-benzothiazol-7-yl)ethyl)amino}ethyl)-3-[2-(1-naphthyl)ethoxy]propanamide**

A solution of dimethylsulfoxide (0.097 g) in dichloromethane (1 mL) was added to a solution of oxalyl chloride (0.079 g) in dichloromethane (10 mL) at -78˚C. The reaction was stirred for 15 minutes and then a solution of *N*-(2-diethylaminoethyl)-*N*-(2-hydroxyethyl)-3-[2-(1-naphthyl)ethoxy]propanamide (0.22 g) in dichloromethane (1 mL+ 1 mL wash) was added and the reaction mixture stirred for a further 15 minutes. Triethylamine (0.29 g) was added and the reaction allowed to warm to room temperature over I hour, the mixture was subsequently diluted (dichloromethane 30 mL), the organics washed with sodium bicarbonate (20 mL), brine (20 mL), dried over anhydrous magnesium sulphate, filtered and concentrated *in vacuo* to give the sub-titled compound (0.21 g).
The crude product was dissolved in methanol (10 mL) and 7-(2-aminoethyl)-4-hydroxy-1,3-benthiazol-2 (3H)-one hydrochloride (prepared according to the procedure outlined in Organic Process Research & Development 2004, 8(4), 628-642; 0.131 g) was added along with acetic acid (0.1 mL) and water (0.1 mL). After stirring at room temperature for 30 minutes, sodium cyanoborohydride (0.020 g) was added and the reaction mixture was stirred overnight. Ammonia (7N in methanol, 1 mL) was added and the mixture was concentrated. The crude residue was purified by flash column chromatography eluting with 1% ammonia; 5%-7% methanol in dichloromethane. The crude product was used directly in the next step.

**e) *N*-[2-(Diethylamino)ethyl]-*N*-(2-{]2-(4-hydroxy-2-oxo-2,3-dihydro-1,3-benzothiazol-7-yl)ethyl]amino}ethyl)-3-[2-(1-naphthyl)ethoxy]propanamide dihydrobromide**

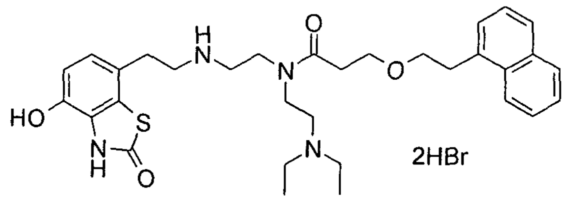

*N*-[2-(Diethylamino) ethyl]-*N*-(2-{[2-(4-hydroxy-2-oxo-2,3-dihydro-1,3-benzothiazol-7-yl) ethyl] amino} ethyl)-3-[2-(1-naphthyl)ethoxy]propanamide (0.052 g) was dissolved in ethanol (1.5 mL) and treated with 48 % hydrobromic acid (21 μl). The white solid dihydrobromide salt (0.058 g) was collected by filtration.

MS: APCI(+ve) 579 (M+1)

$^1$H NMR δ(DMSO) 11.78-11.71 (m, 1H), 10.11-10.06 (m, 1H), 9.51-9.43 (m, 0.33H), 9.21-9.13 (m, 0.66H), 8.75-8.66 (m, 1H), 8.59-8.51 (m, 1H), 8.06 (d, 1H), 7.95-7.90 (m,

I H), 7.79 (d, 1H), 7.60-7.48 (m, 2H), 7.47-7.39 (m, 2H), 6.87 (t, 1H), 6.76 (dd, 1H), 3.78-3.53 (m, 10H), 3.25-3.09 (m, 10H), 2.91-2.80 (m, 2H), 2.73-2.61 (m, 2H), 1.26-1.15 (m, 6H). NMR indicates approximately 2:1 mixture of rotamers at 298K.

**Preparation 2**

***N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide**

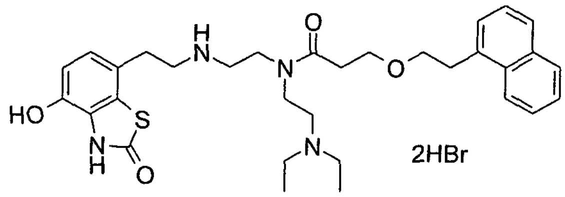

**a) *N'*-(2,2-Dimethoxyethyl)-*N,N*-diethyl-ethane-1,2-diamine.**

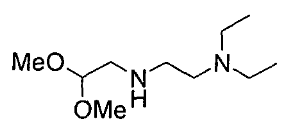

A solution of N,N-diethyl-ethylenediamine (150 g) in methanol (500 mL) was treated dropwise rapidly with glyoxal dimethylacetal (60wt% soln. in water, 225 g) at 10-15˚C. After the addition was complete the solution was warmed to 15˚C, then to 22˚C and left at this temperature for 16 hours. The reaction mixture was treated with 5% palladium on carbon (Johnson-Matthey type 38H paste, 15 g) and hydrogenated at 6 bar until the reaction was complete as judged by GC/MS. The catalyst was removed by filtration and the filtrate evaporated to dryness (toluene azeotrope, 2.5 L), affording 196.2 g of the subtitled compound.

$^1$H NMR ($CDCl_3$): 4.48 (t, 1H), 3.39 (s, 6H), 2.75 (d, 2H), 2.69 (t, 2H), 2.57-2.48 (m, 6H), 1.01 (ts, 6H).

**b) *N*-[2-(Diethylamino)ethyl]-*N*-(2,2-dimethoxyethyl)-3-[2-(1-naphthyl)ethoxy]propanamide.**

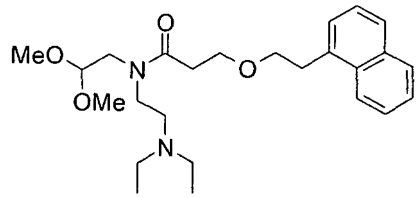

Oxalyl chloride (151 mL) was added dropwise over 45 minutes to a solution of 3-[2-(1-naphthyl)ethoxy]propanoic acid (389 g) (Example 7 step b)) in dichloromethane (2.1 L) and DMF (0.5 mL). The reaction mixture was stirred for a further 16 hours. The mixture was subsequently concentrated, redissolved in DCM (1.7 L) and added dropwise over 1.75 hours at 0˚C to a solution of *N'*-(2,2-dimethoxyethyl)-*N*,*N*-diethylethane-1,2-diamine (325 g) and isopropyldiethylamine (551 mL) in DCM (1.7 L). The resulting mixture was stirred at room temperature for 3 hours, washed with aqueous saturated sodium bicarbonate solution (5x1 L), water (1.5 L) and dried over sodium sulphate and concentrated to give 650 g of the sub-titled compound. m/e 431 ($M+H^+$, 100%)

**c) *N*-[2-(Diethylamino)ethyl]-3-[2-(1-naphthyl)ethoxy]-*N*-(2-oxoethyl)propanamide.**

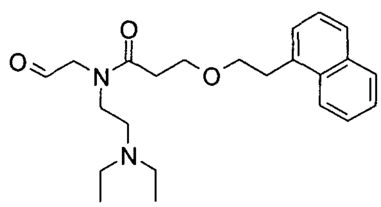

A solution of *N*-[2-(diethylamino)ethyl]-*N*-(2,2-dimethoxyethyl)-3-[2-(1-naphthyl)ethoxy]propanamide (93 g) in DCM (270 mL) was treated dropwise at 0˚C with trifluoroacetic acid (270 mL) over 1.5 hours. After the addition the reaction mixture was allowed to warm to room temperature and stirred for a further 1 hour. The reaction mixture was concentrated and the residue poured into aqueous saturated sodium bicarbonate solution (1800 mL, *caution*). The aqueous mixture was extracted with DCM (4x400 mL) and the combined extracts were dried over magnesium sulphate and concentrated. The residue was used directly in the following reaction.

**d) *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide.**

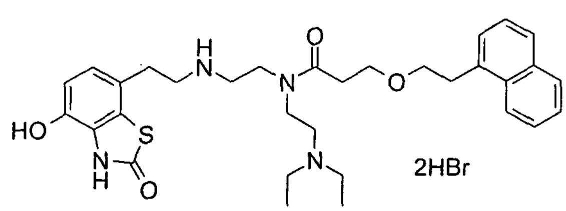

A suspension of 7-(2-amino-ethyl)-4-hydroxy-3H-benzothiazol-2-one hydrochloride (53g) in dry NMP (216 mL) was heated to 60˚C and treated in one portion with a solution of NaOH (8.2 g) in methanol (102 mL). The bright orange suspension was cooled to room temperature and treated dropwise with a solution of *N*-[2-(diethylamino)ethyl]-3-[2-(1-naphthyl)ethoxy]-*N*-(2-oxoethyl)propanamide in dichloromethane (475 mL) over 20 minutes. The reaction was left to stir for 25 minutes. Sodium triacetoxyborohydride (91.5 g) was then added in portions over 20 minutes and the mixture stirred for a further 50 minutes. The reaction mixture was poured into water (1.8 L) and the acidic solution (pH5) was washed with tert. butyl methyl ether (TBME) (3x500 mL). The aqueous phase was basified to pH8 by the addition of solid potassium carbonate and extracted with dichloromethane (3x750 mL); the combined organic extracts were dried

over magnesium sulphate and concentrated to give a dark oil. This was dissolved in ethanol (200 mL) and 48% aqueous hydrobromic acid (73 mL) was added. The solution was aged for 30 minutes then evaporated to dryness. The residue was triturated with ethanol (560 mL); the resultant solid was collected by filtration and dried in vacuo at 50˚C. The sticky solid was suspended in boiling ethanol (100 mL) and filtered while hot. The collected solid was dried in vacuo at 50˚C. This material was recrystallised from ethanol/water (3:1, 500 mL). After standing overnight the resultant solid was collected by filtration and washed with ice-cold ethanol (75 mL). Drying in vacuo at 50˚C for 24hr afforded 57g of the title compound.

Alternative salts of *N*-[2-(Diethylamino)ethyl]-*N*-(2-{[2-(4-hydroxy-2-oxo-2,3-dihydro-1,3-benzothiazol-7-yl) ethyl]amino}ethyl)-3-[2-(1-naphthyl)ethoxy]propanamide can be prepared by mixing a suitable acid with *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide in a solvent. Examples are provided below.

**Adrenergic β2 mediated cAMP production**

Cell preparation

H292 cells were grown in 225cm2 flasks incubator at 37˚C, 5% $CO_2$ in RPMI medium containing, 10% (v/v) FBS (foetal bovine serum) and 2 mM L-glutamine.

Experimental Method

Adherent H292 cells were removed from tissue culture flasks by treatment with Accutase™ cell detachment solution for 15 minutes. Flasks were incubated for 15 minutes in a humidified incubator at 37˚C, 5% $CO_2$. Detached cells were re-suspended in RPMI media (containing 10% (v/v) FBS and 2 mM L-glutamine) at 0.05 x $10^6$ cells per mL. 5000 cells in 100 μL were added to each well of a tissue-culture-treated 96-well plate and the cells incubated overnight in a humidified incubator at 37˚C, 5% $CO_2$. The culture media was removed and cells were washed twice with 100 μL assay buffer and replaced with 50 μL assay buffer (HBSS solution containing 10mM HEPES pH7.4 and 5 mM glucose). Cells were rested at room temperature for 20 minutes after which time 25 μL of rolipram (1.2 mM made up in assay buffer containing 2.4% (v/v) dimethylsulphoxide) was added. Cells were incubated with rolipram for 10 minutes after which time Compound A was added and the cells were incubated for 60 minutes at room temperature. The final rolipram concentration in the assay was 300 μM and final vehicle concentration was 1.6% (v/v) dimethylsulphoxide. The reaction was stopped by removing supernatants, washing once with 100 μL assay buffer and replacing with 50 μL lysis buffer. The cell monolayer was frozen at -80˚C for 30 minutes (or overnight).

AlphaScreen™ cAMP detection

The concentration of cAMP (cyclic adenosine monophosphate) in the cell lysate was determined using AlphaScreen™ methodology. The frozen cell plate was thawed for 20 minutes on a plate shaker then 10 μL of the cell lysate was transferred to a 96-well white plate. 40 μL of mixed AlphaScreen™ detection beads pre-incubated with biotinylated cAMP, was added to each well and the plate incubated at room temperature for 10 hours in the dark. The AlphaScreen™ signal was measured using an EnVision spectrophotometer (Perkin-Elmer Inc.) with the recommended manufacturer's settings. cAMP concentrations were determined by reference to a calibration curve determined in the same experiment using standard cAMP concentrations. A concentration response curve for Compound A was constructed and data was fitted to a four parameter logistic equation to determine both the $pEC_{50}$ and Intrinsic Activity. Intrinsic Activity was expressed as a fraction relative to the maximum activity determined for formoterol in each experiment. A result for Compound A is in Table 1.

**Selectivity Assays**

**Adrenergic α1D**

Membrane Preparation

Membranes were prepared from human embryonic kidney 293 (HEK293) cells expressing recombinant human $\alpha l_D$ receptor. These were diluted in Assay Buffer (50mM HEPES, 1mM EDTA, 0.1% gelatin, pH 7.4) to provide a final concentration of membranes that gave a clear window between maximum and minimum specific binding.

Experimental Method

Assays were performed in U-bottomed 96-well polypropylene plates. 10 μL [$^3$H]-prazosin (0.3 nM final concentration) and 10 μL of Compound A (10x final concentration) were added to each test well. For each assay plate 8 replicates were obtained for [$^3$H]-prazosin binding in the presence of 10 μL vehicle (10% (v/v) DMSO in Assay Buffer; defining maximum binding) or 10μL BMY7378 (10 μM final concentration; defining non-specific binding (NSB)). Membranes were then added to achieve a final volume of 100 μL. The plates were incubated for 2 hours at room temperature and then filtered onto PEI coated GF/B filter plates, pre-soaked for 1 hour in Assay Buffer, using a 96-well plate Tomtec cell harvester. Five washes with 250 μL wash buffer (50mM HEPES, 1mM EDTA, pH 7.4) were performed at 4°C to remove unbound radioactivity. The plates were dried then sealed from underneath using Packard plate sealers and MicroScint-O (50 μL) was added to each well. The plates were sealed (TopSeal A) and filter-bound radioactivity was measured with a scintillation counter (TopCount, Packard BioScience) using a 3-minute counting protocol.

Total specific binding ($B_0$) was determined by subtracting the mean NSB from the mean maximum binding. NSB values were also subtracted from values from all other wells. These data were expressed as percent of $B_0$. Compound concentration-effect curves (inhibition of [$^3$H]-prazosin binding) were determined using serial dilutions typically in the range 0.1 nM to 10 μM. Data was fitted to a four parameter logistic equation to determine the compound potency, which was expressed as pIC50 (negative log molar concentration inducing 50% inhibition of [$^3$H]-prazosin binding). Result is shown in Table I below.

**Adrenergic β1**

Membrane Preparation

Membranes containing recombinant human adrenergic beta 1 receptors were obtained from Euroscreen. These were diluted in Assay Buffer (50mM HEPES, 1mM EDTA, 120mM NaCl, 0.1% gelatin, pH 7.4) to provide a final concentration of membranes that gave a clear window between maximum and minimum specific binding.

Experimental Method

Assays were performed in U-bottomed 96-well polypropylene plates. 10 μL [$^{125}$I]-Iodocyanopindolol (0.036 nM final concentration) and 10 μL of Compound A (10x final concentration) were added to each test well. For each assay plate 8 replicates were obtained for [$^{125}$I]-Iodocyanopindolol binding in the presence of 10 μL vehicle (10% (v/v) DMSO in Assay Buffer; defining maximum binding) or 10 μL Propranolol (10 μM final concentration; defining non-specific binding (NSB)). Membranes were then added to achieve a final volume of 100 μL. The plates were incubated for 2 hours at room temperature and then filtered onto PEI coated GF/B filter plates, pre-soaked for 1 hour in Assay Buffer, using a 96-well plate Tomtec cell harvester. Five washes with 250 μL wash buffer (50mM HEPES, 1mM EDTA, 120mM NaCl, pH 7.4) were performed at 4°C to remove unbound radioactivity. The plates were dried then sealed from underneath using Packard plate sealers and MicroScint-O (50 μL) was added to each well. The plates were sealed (TopSeal A) and filter-bound radioactivity was measured with a scintillation counter (TopCount, Packard BioScience) using a 3-minute counting protocol.

Total specific binding ($B_0$) was determined by subtracting the mean NSB from the mean maximum binding. NSB values were also subtracted from values from all other wells. These data-were expressed as percent of $B_0$. Compound concentration-effect curves (inhibition of [$^{125}$I]-Iodocyanopindolol binding) were determined using serial dilutions typically in the range 0.1 nM to 10 μM. Data was fitted to a four parameter logistic equation to determine the compound potency, which was expressed as $pIC_{50}$ (negative log molar concentration inducing 50% inhibition of [$^{125}$I]-Iodocyanopindolol binding). A result is shown in Table 1 below.

**Dopamine D2**

Membrane Preparation

Membranes containing recombinant human Dopamine Subtype D2s receptors were obtained from Perkin Elmer. These were diluted in Assay Buffer (50mM HEPES, 1mM EDTA, 120mM NaCl, 0.1% gelatin, pH 7.4) to provide a final concentration of membranes that gave a clear window between maximum and minimum specific binding.

Experimental Method

Assays were performed in U-bottomed 96-well polypropylene plates. 30 μL [$^3$H]-spiperone (0.16 nM final

concentration) and 30 μL of Compound A (10x final concentration) were added to each test well. For each assay plate 8 replicates were obtained for [$^3$H]-spiperone binding in the presence of 30 μL vehicle (10% (v/v) DMSO in Assay Buffer; defining maximum binding) or 30 μL Haloperidol (10 μM final concentration; defining non-specific binding (NSB)). Membranes were then added to achieve a final volume of 300 μL. The plates were incubated for 2 hours at room temperature and then filtered onto PEI coated GF/B filter plates, pre-soaked for 1 hour in Assay Buffer, using a 96-well plate Tomtec cell harvester. Five washes with 250 μL wash buffer (50mM HEPES, 1 mM EDTA, 120mM NaCl, pH 7.4) were performed at 4°C to remove unbound radioactivity. The plates were dried then sealed from underneath using Packard plate sealers and MicroScint-O (50 μL) was added to each well. The plates were sealed (TopSeal A) and filter-bound radioactivity was measured with a scintillation counter (TopCount, Packard BioScience) using a 3-minute counting protocol.

Total specific binding ($B_0$) was determined by subtracting the mean NSB from the mean maximum binding. NSB values were also subtracted from values from all other wells. These data were expressed as percent of Bo. Compound concentration-effect curves (inhibition of [$^3$H]-spiperone binding) were determined using serial dilutions typically in the range 0.1 nM to 10 μM. Data was fitted to a four parameter logistic equation to determine the compound potency, which was expressed as $pIC_{50}$ (negative log molar concentration inducing 50% inhibition of [$^3$H]-spiperone binding). A result for Compound A is shown in Table 1.

Table 1

| Compound | β2 pEC50 | β2 Int Act | α1 bind pIC50 | β1 bind p IC50 | D2 bind pIC50 |
|---|---|---|---|---|---|
| A | 8.2 | 0.8 | 6.6 | <5 | 6.1 |

The present invention will now be further explained by reference to the following illustrative Examples. Certain of the Examples refer to the following Figure:

Figure 1. Methacholine- induced bronchoconstriction in the guinea pig. Guinea pigs were dosed with either vehicle, 1 μg/kg and 27μg/kg Compound A, 0.03μg/kg tiotropium bromide or a combination of 1 μg/kg Compound A and 0.03 μg/kg tiotropium bromide 2 hours prior to methacholine challenge.

Example 1

**Evaluation of compound activity on intra-alveolar neutrophil migration after aerosol challenge with lipopolysaccharride (LPS) in the guinea-pig.**

Male Dunkin-Hartley guinea-pigs (300-600g) are placed into open fronted guinea-pig holding cones attached at random around a cylindrical aerosol chamber. Guinea-pigs are held in the challenge cones and exposed to an aerosol of vehicle, or LPS at concentrations of 0.1-30μg/ml in 0.9%saline per group. Aerosols are generated using 2 jet nebulisers per column with a flow rate of 12 L/m. 10ml of the challenge agent is placed into each nebuliser. Alternatively animals receive an intratracheal dose of 0.1-10μg/kg. This is repeated up to 8 times according to the experimental protocol.

Guinea-pigs are dosed with vehicle, standard compound or test compound by the appropriate route and frequency at various time points before and after challenge depending upon the experimental protocol. Test compound groups could either be the same compound at different doses or single doses of different compounds or a combination of the two. Test compounds are given by intraperitoneal, intravenous or subcutaneous injection or by inhalation or intratracheal administration. Challenged guinea-pigs are killed by anaesthesia overdose (0.5ml Euthetal i.p.) at 4h-24h post challenge. The lungs are then lavaged. After the trachea is exposed and cannulated using a luer fitting cannula (orange =size 8FG), the lungs are lavaged with 3 x 5ml aliquots of Hanks Buffered Salt Solution (HBSS, EDTA -free). The lavaging is performed with gentle massaging of the chest to ensure appropriate agitation of the fluid in the lungs. The washes are harvested into a 15ml conical, polypropylene centrifuge tube, an aliquot of BAL fluid is removed and counted on Sysmex (Sysmex UK, Milton Keynes). Cytospin slides are prepared by adding a 100 μl aliquot of BAL fluid into cytospin funnels in a Shandon Cytospin3 operated at 700 rpm for 5 min. Slides are stained on the Hema-Tek-2000 automatic slide stainer, using Wright-Giemsa stain and typically, 200 cells are counted under a microscope. Cells are classified as eosinophils, neutrophils and mononuclear cells (mononuclear cells included monocytes, macrophages and lymphocytes) and are expressed as a percentage of the total count.

Example 2

**Evaluation of compound activity on intra-alveolar neutrophil migration after aerosol challenge with lipopolysaccharride (LPS) in the mouse.**

Male C57BL/6/J or BALB/C mice (20-35g) are placed in Perspex exposure boxes in groups of up to 20 and exposed to an aerosol of either 0.3 mg/ml LPS or 0.9% w/v saline. The LPS (Sigma, E.Coli, Ref L-3755, Serotype 026: B6, Lot no. 111k4078) is made up in 0.9% w/v saline. An aerosol is generated using two jet nebulisers operated at a flow rate of 12 L/min (6L/min for each nebuliser) for 15 min. Alternatively animals receive an intratracheal dose of 0.1-10μg/kg. This may be repeated up to 8 times.

Mice are dosed with vehicle, standard compound or test compound by the appropriate route and frequency at various time points before and after challenge depending upon the experimental protocol. Test compound groups could either be the same compound at different doses or single doses of different compounds or a combination of the two. Test compounds are given by intraperitoneal, intravenous or subcutaneous injection or by inhalation or intratracheal administration.

Mice are killed with an overdose of Euthatal i.p 30 minutes, 1-24hr after LPS challenge. When circulation has ceased, the trachea is cannulated (Portex intravenous cannula) and the airways lavaged with 3 x 0.3ml of Isoton II (Beckman Coulter Ref. 8448011 Lot no.25775). For cytospins, 100μl of the BALF is added to a cytospin funnel and spun, using a ThermoShandon Cytospin model 3 or 4, at 700 rpm for 5 min. Cells on the slide are stained on the Hema-Tek-2000 automatic slide stainer, using Wright-Giemsa stain and differential cell counts carried out to differentiate eosinophils, neutrophils and lymphomononuclear cells (including monocytes, macrophages and lymphocytes). Typically, 200 cells are counted per slide and each cell type expressed as a percentage of the total count. BALF total white cell count is measured using a Sysmex (Sysmex UK, Milton Keynes).

Example 3

**Evaluation of lung function in anaesthetised guinea-pigs.**

Male Dunkin-Hartley guinea pigs (300-600g) were weighed and dosed with vehicle (0.05M phosphate, 0.1% Tween 80, 0.6% saline, pH 6) or compound *via* the intratracheal route under recoverable gaseous anaesthesia (5% halothane in oxygen). Animals were dosed with compound or vehicle two hours prior to the administration of histamine or methacholine.

Guinea pigs were anaesthetised with pentobarbitone (1 mL/kg of 60 mg/mL solution *i.p.*) approximately 30 minutes prior to the first bronchoconstrictor administration. The trachea was cannulated and the animal ventilated using a constant volume respiratory pump (Harvard Rodent Ventilator model 683) at a rate of 60 breath/min and a tidal volume of 5 ml/kg. A jugular vein was cannulated for the administration of histamine, methacholine or maintenance anaesthetic (0.1 mL of pentobarbitone Solution, 60 mg/mL, as required).

The animals were transferred to a Flexivent System (SCIREQ, Montreal, Canada) in order to measure airway resistance. The animals were ventilated (quasi-sinusoidal ventilation pattern) at 60 breaths/min at a tidal volume of 5 mL/kg. A positive end expiratory pressure of 2-3 cm$H_2O$ was applied. Respiratory resistance was measured using the Flexivent "snapshot" facility (1 second duration, 1 Hz frequency). Once stable baseline resistance value had been obtained the animals were given histamine or methacholine in ascending doses (0.5, 1, 2, 3 and 5μg/kg, *i.v*) at approximately 4-minute intervals *via* the jugular catheter. After each administration of bronchoconstrictor the peak resistance value was recorded. Guinea pigs were euthanised with approximately 1.0mL pentobarbitone sodium (Euthatal) intravenously after the completion of the lung function measurements. Percentage bronchoprotection produced by a compound was calculated at each dose of brochoconstrictor as follows:

$$\%\, bronchoprotection = \frac{\%\, changeR_{veh} - \%\, changeR_{cmpd}}{\%\, changeR_{veh}}$$

Where % change $R_{veh}$ is the mean of the maximum percentage change in airway resistance in the vehicle treated group. The results reported were measured after 5μg/kg histamine or methacholine and were expressed as percentage bronchoprotection (mean ± s.e.mean).

Compound A and tiotropium bromide combination:

Guinea pigs dosed with vehicle, 1 and 27 μg/kg Compound A, 0.03 μg/kg tiotropium bromide or a combination of 1 μg/kg Compound A and 0.03 μg/kg tiotropium bromide *via* the intratracheal route. Administration of increasing intravenous doses of methacholine (0.5, 1, 2, 3 and 5 μg/kg) evoked dose-related bronchoconstriction in the vehicle treated animals ranging from 7.8±4.1% at 0.5 μg/kg to 1898±211% at 5 μg/kg two hours after vehicle administration (n=9). Intratracheal administration of tiotropium bromide at 0.03 μg/kg produced 13% inhibition of methacholine-induced bronchoconstriction (1656±269% increase in resistance, n = 8). Intratracheal administration of compound A (1 and 27μg/kg) produced 15 and 82% inhibition of methacholine-induced bronchoconstriction (1619±235 and 347±71% increase in resistance, respectively; n=8 and 6, respectively). The combination of tiotropium bromide (0.03 μg/kg) and compound A (1 μg/kg) produced 43% inhibition of methacholinle-induced bronchoconstriction (1087±123% increase in resistance; n=8) (Figure 1).

Example 4

**Evaluation of Compounds on Antigen induced Eosinophilia in Ovalbumin Sensitised Brown Norway Rats.**

On day 0 of the study Brown Norway rats are given a subcutaneous injection of 500 μg ovalbumin adsorbed onto 100 mg aluminium hydroxide in 0.4 mL saline in two distinct sites, approximately 0.2 mL per site. Day 14 and 15 following sensitisation the rats are challenged with aerosolised ovalbumin for 15 minutes. The rats are placed in groups of 10 in an acrylic box (internal dimensions 320mm wide x 320mm deep x 195 mm high, 20L volume). 8mL of 10 mg/mL ovalbumin in 0.9% saline, or 0.9% saline alone, is placed in each of two jet nebulizers (Sidestream®, Profile Respiratory Systems Ltd.). Compressed air at 6 L/min is passed through each nebulizer and the output of the nebulizers is passed into the box containing the rats.

Rats are dosed via the appropriate route with vehicle, standard compound or test compound at various time points before and after challenge depending upon the experimental protocol. Rats are euthanised with 0.5 mL pentobarbitone sodium (Euthatal) intraperitoneally at various times after challenge. A tracheotomy is performed and the trachea cannulated. The airway is then lavaged using 3 mL sterile PBS at room temperature. The PBS is left in the airway for 10 seconds before being removed. The PBS containing cells is placed into a 15 mL centrifuge tube on ice. This process is repeated three times. The final volume recovered is recorded. An aliquot of BAL fluid is removed and counted using a Sysmex (Sysmex UK, Milton Keynes).

Cytospin slides are prepared by adding a 100 μl aliquot of BAL fluid into cytospin funnels in a Shandon Cytospin 3 operated at 700 rpm for 5 min. Slides are stained on the Hema-Tek-2000 automatic slide stainer, using Wright-Giemsa stain and typically, 200 cells are counted under a microscope. Cells are classified as eosinophils, neutrophils and mononuclear cells. Mononuclear cells included monocytes, macrophages and lymphocytes.

Example 5

**Evaluation of Compounds on Antigen induced eosinophilia in ovalbumin sensitised mice.**

20-25g male BALB/c mice are sensitized to ovalbumin by i.p administration of 100 μg of grade V ovalbumin (Sigma) adsorbed onto 1mg of aluminium hydroxide gel mixture (Fisher Scientific UK) in 0.3 ml saline. Groups of mice are pre-dosed with compound if required, a minimum oftwo weeks after sensitization. They are then dosed daily for 1-8 days as study protocol specified, with test compound or 0.25 ml vehicle.

Each day of the 1-8 days, 1 hour after dosing, the mice are placed in perspex chambers (20x11x11cm, 10 mice max./chamber) and administered an aerosol challenge of 20mg $ml^{-1}$ ovalbumin for 36 min (8 ml for 18 min followed by another 8 ml for 18 min). Aerosol delivery is achieved using a DeVilbiss jet nebulizer with a flow rate of 61 $min^{-1}$. 24h after the last dose the mice are killed with euthatal 0.2 ml i.p. and blood samples are taken (in EDTA tubes) for differential cell count analysis, the trachea is cannulated using a pink luer mount Portex cannula cut to 1 cm and the lungs are lavaged using 3 washes of 1ml of Isoton II.. For cytospins, 100μl of the BALF is added to a cytospin funnel and spun, using a ThermoShandon Cytospin model 3 or 4, at 700 rpm for 5 min. Cells on the slide are stained on the Hema-Tek-2000 automatic slide stainer, using Wright-Giemsa stain and differential cell counts carried out to differentiate eosinophils, neutrophils and lymphomononuclear cells (including monocytes, macrophages and lymphocytes). Typically, 200 cells are counted per slide and each cell type expressed as a percentage of the total count. BALF total white cell count is measured using a Sysmex (Sysmex UK, Milton Keynes).

Example 6

**Evaluation on the effect of compound on lung function and BAL-neutrophilia following acute smoke exposure in the mouse**

BALB/c or C57BL6/J mice undergo whole body exposure to main stream smoke (50 min/12 cigarettes) and fresh air once or twice a day for 1-9 days. Mice are dosed via the appropriate route with vehicle, standard compound or test compound at various time points before and after challenge depending upon the experimental protocol. On the final day of the experiment, mice are either killed with euthatal 0.2 ml i.p. and broncho-aveolar lavage fluid obtained for analysis of white blood cell infiltration (as described above) or lung function is assessed using a Flexivent System (SCIREQ, Montreal, Canada). Alternatively lung mechanics are measured using a forced manoeuvres system (EMMS).
Mice are anaesthetised with pentobarbitone (1/10dilution at a dose volume of 1 mL/kg intraperitoneally). The trachea is cannulated and the animal transferred to the Flexivent System where they are ventilated (quasi-sinusoidal ventilation pattern) at a rate of 150 breath/min and a tidal volume of 10 ml/kg in order to measure airways resistance. Respiratory resistance is measured using the Flexivent "snapshot" facility (1 second duration, 1 Hz frequency). Mice are euthanised with approximately 0.5mL pentobarbitone sodium (Euthatal) intravenously after the completion of the lung function measurements.

## Claims

**1.** A pharmaceutical product comprising, in combination, a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide, and a second active ingredient which is a muscarinic antagonist which is Aclidinium bromide, Glycopyrrolate, Oxitropium bromide, Pirenzepine, telenzepine or Tiotropium bromide.

**2.** A kit comprising:

a preparation of a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide,
a preparation of a second active ingredient which is a muscarinic antagonist which is Aclidinium bromide, Glycopyrrolate, Oxitropium bromide, Pirenzepine, telenzepine or Tiotropium bromide, and optionally,
instructions for the simultaneous, sequential or separate administration of the preparations to a patient in need thereof.

**3.** A pharmaceutical composition comprising, in admixture:

a first active ingredient which is *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide dihydrobromide;
a second active ingredient which is a muscarinic antagonist which is Aclidinium bromide, Glycopyrrolate, Oxitropium bromide, Pirenzepine, telenzepine or
Tiotropium bromide; and,
a pharmaceutically acceptable adjuvant, diluent or carrier.

**4.** A pharmaceutical product, kit or composition as claimed in any one of the preceding claims wherein the second active ingredient is Tiotropium bromide.

**5.** A pharmaceutical product, kit or composition as claimed in any one of claims 1-4 for use in therapy.

**6.** Use of a pharmaceutical product, kit or composition as claimed in any one of claims 1-4 in the manufacture of a medicament for the treatment of a respiratory disease.

**7.** A pharmaceutical product, kit or composition as claimed in any one of claims 1-4 for use in the treatment of a respiratory disease.

**8.** Use according to claim 6 or 7, wherein the respiratory disease is chronic obstructive pulmonary disease or asthma.

## Patentansprüche

1. Pharmazeutisches Produkt, enthaltend, in einer Kombination, einen ersten Wirkstoff, bei dem es sich um *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]-propanamiddihydrobromid handelt, und einen zweiten Wirkstoff, bei dem es sich um einen muskarinischen Antagonisten handelt, bei dem es sich um Aclidiniumbromid, Glykopyrrolat, Oxitropiumbromid, Pirenzepin, Telenzepin oder Tiotropiumbromid handelt.

2. Kit, enthaltend:

   eine Zubereitung eines ersten Wirkstoffs, bei dem es sich um *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamiddihydrobromid handelt,
   eine Zubereitung eines zweiten Wirkstoffs, bei dem es sich um einen muskarinischen Antagonisten handelt, bei dem es sich um Aclidiniumbromid, Glykopyrrolat, Oxitropiumbromid, Pirenzepin, Telenzepin oder Tiotropiumbromid handelt, und gegebenenfalls
   Anweisungen für die gleichzeitige, aufeinanderfolgende oder getrennte Verabreichung der Zubereitungen an einen deren bedürftigen Patienten.

3. Pharmazeutische Zusammensetzung, enthaltend, als Mischung:

   einen ersten Wirkstoff, bei dem es sich um *N*-[2-(Diethylamino)ethyl]-*N*-(2-{[2-(4-hydroxy-2-oxo-2,3-dihydro-1,3-benzothiazol-7-yl)ethyl]amino}-ethyl)-3-[2-(1-naphthyl)ethoxy]propanamiddihydrobromid handelt;
   einen zweiten Wirkstoff, bei dem es sich um einen muskarinischen Antagonisten handelt, bei dem es sich um Aclidiniumbromid, Glykopyrrolat, Oxitropiumbromid, Pirenzepin, Telenzepin oder Tiotropiumbromid handelt; und
   ein pharmazeutisch annehmbares Adjuvans, ein pharmazeutisch annehmbares Verdünnungsmittel oder einen pharmazeutisch annehmbaren Träger.

4. Pharmazeutisches Produkt, Kit oder Zusammensetzung nach einem der vorhergehenden Ansprüche, bei dem es sich bei dem zweiten Wirkstoff um Tiotropiumbromid handelt.

5. Pharmazeutisches Produkt, Kit oder Zusammensetzung nach einem der Ansprüche 1 - 4 zur Verwendung in der Therapie.

6. Verwendung eines pharmazeutischen Produkts, eines Kits oder einer Zusammensetzung nach einem der Ansprüche 1 -4 bei der Herstellung eines Medikaments zur Behandlung einer Atemwegserkrankung.

7. Pharmazeutisches Produkt, Kit oder Zusammensetzung nach einem der Ansprüche 1 - 4 zur Verwendung bei der Behandlung einer Atemwegserkrankung.

8. Verwendung nach Anspruch 6 oder 7, wobei es sich bei der Atemwegserkrankung um chronische obstruktive Atemwegserkrankung oder Asthma handelt.

## Revendications

1. Produit pharmaceutique comprenant, en combinaison, un premier principe actif qui est le dibromhydrate de *N*-[2-(diéthylamino)éthyl]-*N*-(2-{[2-(4-hydroxy-2-oxo-2,3-dihydro-1,3-benzothiazol-7-yl)éthyl]amino}éthyl)-3-[2-(1-naphtyl)éthoxy]propanamide, et un deuxième principe actif qui est un antagoniste muscarinique qui est le bromure d'aclidinium, le glycopyrrolate, le bromure d'oxitropium, la pirenzépine, la telenzépine ou le bromure de tiotropium.

2. Kit comprenant :

   une préparation d'un premier principe actif qui est le dibromhydrate de *N*-[2-(diéthylamino)éthyl] - *N*-(2-{[2-(4-hydroxy-2-oxo-2,3-dihydro-1,3-benzothiazol-7-yl)éthyl]amino}éthyl)-3-[2-(1-naphtyl)éthoxy]propanamide,
   une préparation d'un deuxième principe actif qui est un antagoniste muscarinique qui est le bromure d'aclidinium, le glycopyrrolate, le bromure d'oxitropium, la pirenzépine, la telenzépine ou le bromure de tiotropium, et éventuellement,
des instructions pour l'administration simultanée, séquentielle ou séparée des préparations à un patient en ayant besoin.

**3.** Composition pharmaceutique comprenant, en mélange :

un premier principe actif qui est le dibromhydrate de *N*-[2-(diéthylamino)éthyl]-*N*-(2-{[2-(4-hydroxy-2-oxo-2,3-dihydro-1,3-benzothiazol-7-yl)éthyl]amino}éthyl)-3-[2-(1-naphtyl)éthoxy]propanamide ;
un deuxième principe actif qui est un antagoniste muscarinique qui est le bromure d'aclidinium, le glycopyrrolate, le bromure d'oxitropium, la pirenzépine, la telenzépine ou le bromure de tiotropium ; et,
un adjuvant, un diluant ou un support pharmaceutiquement acceptable.

**4.** Produit pharmaceutique, kit ou composition pharmaceutique selon l'une quelconque des revendications précédentes, **caractérisés en ce que** le deuxième principe actif est le bromure de tiotropium.

**5.** Produit pharmaceutique, kit ou composition pharmaceutique selon l'une quelconque des revendications 1 à 4, destinés à être utilisés en thérapie.

**6.** Utilisation d'un produit pharmaceutique, d'un kit ou d'une composition pharmaceutique selon l'une quelconque des revendications 1 à 4, dans la fabrication d'un médicament destiné au traitement d'une maladie respiratoire.

**7.** Produit pharmaceutique, kit ou composition pharmaceutique selon l'une quelconque des revendications 1 à 4, destinés à être utilisés dans le traitement d'une maladie respiratoire.

**8.** Utilisation selon la revendication 6 ou 7, **caractérisée en ce que** la maladie respiratoire est la bronchopneumopathie chronique obstructive ou l'asthme.

FIGURE 1

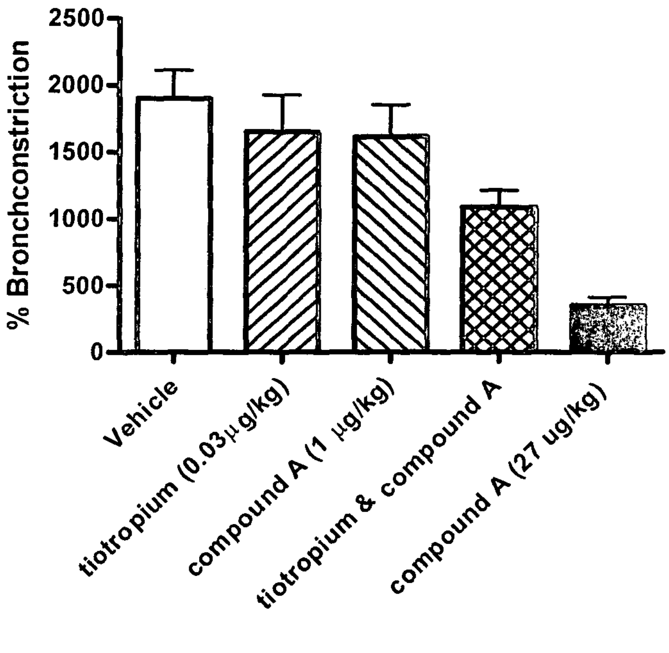
% Bronchconstriction
2500
2000
1500
1000
500
0
Vehicle
tiotropium (0.03μg/kg)
compound A (1 μg/kg)
tiotropium & compound A
compound A (27 ug/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- SE 2006000927 W
- WO 2007018461 A


### Non-patent literature cited in the description


- **Tomlinson et al.** *Thorax,* 2005, vol. 60, 282-287
- *Organic Process Research & Development,* 2004, vol. 8 (4), 628-642